(19) Europäisches Patentamt
European Patent Office
Office européen des brevets

(11) Publication number: 0 234 083 B1

# (12) EUROPEAN PATENT SPECIFICATION

(45) Date of publication of patent specification: 15.04.92

(51) Int. Cl.[5]: G01N 33/546, //C12Q1/00, G01N33/553

(21) Application number: 86305190.0

(22) Date of filing: 04.07.86

## (54) Carrier for a biologically active component for immunoassay or enzymatic reaction.

(30) Priority: 25.02.86 JP 38279/86

(43) Date of publication of application:
02.09.87 Bulletin 87/36

(45) Publication of the grant of the patent:
15.04.92 Bulletin 92/16

(84) Designated Contracting States:
BE DE FR GB IT NL

(56) References cited:
EP-A- 0 007 654
EP-A- 0 038 960
EP-A- 0 176 638
FR-A- 2 450 263
US-A- 4 061 466
US-A- 4 369 226

PATENT ABSTRACTS OF JAPAN, vol. 10, no. 374 (P-527)[2431], 12 December 1986

(73) Proprietor: TOSOH CORPORATION
4560, Oaza-Tonda Shinnanyo-shi
Yamaguchi 746(JP)

(72) Inventor: Ishida, Hiroshi
18-6 Nakamachi 3-chome
Machida-shi Tokyo(JP)
Inventor: Higo, Yuji
3-24 Tenjin-cho Showa-ku
Nagoya-shi Aichi-ken(JP)
Inventor: Inoue, Masuo
1-36-3-1206 Izumihon-cho
Komae-shi Tokyo(JP)

(74) Representative: Sheard, Andrew Gregory et al
Kilburn & Strode 30, John Street
London WC1N 2DD(GB)

EP 0 234 083 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a carrier for a biologically active component for immunoassay or enzymatic reaction, and a process for its preparation. More particularly, the present invention relates to a carrier for a biologically active component for immunoassay wherein a very small amount of a substance in a human body fluid is quantitatively and selectively analyzed by an immunological reaction, or for immobilizing enzymes for the industrial utilization of the enzymes.

The immunoassay is a method for quantitatively analyzing the concentration of antigens or antibodies by taking the advantage of the specificity of the reaction between antigens and the corresponding antibodies, which reaction takes place even at very low concentrations of the reactants.

The immunoassay generally includes two methods, i.e. a sandwich method and a competition method. In many cases, the immunological reagents comprise immobilized or fixed antibodies or antigens, and labelled antigens or antibodies. The rate of immunological reaction between antigens and antibodies is, by its nature, extremely high, and the binding constant of an antigen-antibody complex is extremely large at a level of from $10^7$ to $10^{12}$ ℓ/mol. Therefore, the antigens or antibodies should be analyzed quickly and quantitatively even if their concentration is low. However, in the actual analysis, there are problems in the reaction rate and the quantitative analysis because of practical difficulties such as the difficulty in stirring the immobilized antibody or antigen carrier particles during the reaction, the non-uniformity of the surface area of the carrier particles, or the insufficiency of the removal of the labelled antigens or antibodies which do not form antibody-antigen complexes (hereinafter referred to simply as "B/F separation").

It is therefore an object of the present invention to overcome such problems.

The present invention provides a carrier for a biologically active component for immunoassay or enzymatic reaction, which comprises:

a) a thermoplastic resin bead having an average diameter of from 0.05 to 20 mm,

b) from 1 to 25% by weight, based on the bead, of a magnetically responsive powder bonded to the bead, and

c) a polymer coated thereon in a thickness of from 2 to 30 µm, said polymer having a number average degree of polymerization of from 20 to 5,000 and having, or being capable of having, functional groups capable of binding, or being activated to bind, the biologically active component.

Further, the present invention provides a process for preparing such a carrier, which comprises depositing a magnetically responsive powder on the surface of the thermoplastic resin bead in an amount of from 1 to 25% by weight, based on the bead, and forming a layer of the polymer (c) on the bead in a thickness of from 2 to 30 µm.

Now, the present invention will be described in detail with reference to the preferred embodiments.

In the accompanying drawings, Figure 1 shows the results of the test for detecting ferritin in Example 8.

Figure 2 to 4 show the results of the tests for detecting ferritin, α-fetoprotein (AFP) and human chorionic gonadotropin (HCG), respectively, in Example 10.

Figure 5 is a graph showing the standard curve of L-thyroxine ($T_4$) in a competition method in Example 11.

The bead constituting the core of the carrier of the present invention is made of a thermoplastic resin which includes a polyolefin such as polyethylene or polypropylene, a vinyl resin such as polyvinyl chloride, polystyrene or polyacrylate, a polyester resin such as polyethylene terephathalate, a polyamide resin such as nylon®, a copolymer resin such as an ethylene-vinyl acetate copolymer or a styrene-butadiene copolymer, and a mixture thereof. The thermoplastic resin may further contain fillers. The resin may be pelletted into small sized pellets by a well-known method such as a strand cutting method or an underwater cutting method. It is also possible to use a classified powder of the resin. By using such pellets or powder, beads having a uniform size may be prepared by e.g. extrusion, followed by stretching operation. The beads are preferably substantially spherical. A preferred method for the preparation of such spherical thermoplastic resin beads having a uniform size comprises dispersing a thermoplastic resin having a substantially uniform shape in a medium in which the resin is insoluble, at a temperature lower than the melting point of the resin and then heating the dispersion at a temperature of from the melting point of the resin to a temperature not higher than 30 ° C above the melting point.

The average diameter of the thermoplastic resin beads thereby obtained, is determined by the volume of the shaped starting resin particles to be used.

As a dispersant to be used in the method, there may be mentioned a polymer such as polyvinyl alcohol or polyvinyl pyrrolidone, an inorganic fine powder such as alumina or silica, or a surfactant such as a naphthalene sulfonic acid-formalin condensation product, sodium oleate, dodecylamine, polyoxyethylenealkyl ether or polyoxyethylenealkylphenol ether. The dispersant is used usually within a range of from 0.001

to 5.0% by weight, preferably from 0.01 to 2.0% by weight, relative to the thermoplastic resin. If the dispersant is outside the above range, coagulation of the resin tends to take place even when the heating is controlled, whereby it is difficult to obtain spherical beads. The heating may be conducted at the melting point of the resin, but in order to facilitate the formation of spherical beads, the temperature may be raised to a temperature not higher than 30 ° C, preferably not higher than 20 ° C, above the melting point of the resin.

The medium may be water, an aqueous salt solution, an organic solvent incapable of dissolving the thermoplastic resin, silicon oil, liquid paraffin, a lubricating oil or the like, and it is suitably selected depending upon the melting point of the thermoplastic resin to be treated. The ratio of the medium to the thermoplastic resin may be at any level so long as the thermoplastic resin can be well dispersed in the medium, and the medium is usually in an amount of from 1 to 100% by weight, preferably from 5 to 10% by weight relative to the thermoplastic resin.

The apparatus to be used for the heating and stirring is not required to be of a special type, and may be of a usual type equipped with a jacket or a coil heater and a vane-type stirrer. The dispersed state of the system can be maintained so far as the system is in a flowing state, and can be accomplished by moderate stirring. Thus, smooth spherical beads having a diameter of from 0.05 to 20 mm, preferably from 0.2 to 2.2 mm, can be obtained, The spherical thermoplastic resin beads thus obtained have a uniform surface area and a smooth surface, whereby they can be easily rotated, transferred, dispersed or classified. Therefore, they are useful not only as adsorbents or immobilizing substrates for proteins, but also as supporting substrates for catalysts, magnetic materials or coloring agents.

Then, magnetically responsive powder is deposited on the resin beads. The magnetically responsive powder may be a powder of iron, tri-iron tetroxide, nickel, iron-cobalt, silicon steel or a soft ferrite of the formula $MFe_2O_4$ wherein M is Mn, Zn, Ni, Cd, Cu, Mg, Sr or Ba having an average particle size of from 0.01 to 10 $\mu$m. Such magnetically responsive powder is employed in an amount of from 1 to 25% by weight, preferably from 2 to 15% by weight, based on the thermoplastic resin beads.

The magnetically responsive powder may be deposited on the surface of the thermoplastic resin beads or may be embedded in the beads by heating the thermoplastic resin beads together with the magnetically responsive powder at a temperature within a range of ± 50 ° C, preferably ± 20 ° C of the melting point of the thermoplastic resin. The beads having the magnetically responsive powder thus supported thereon will be hereinafter referred to as "magnetic resin beads".

Then, a polymer is further coated on the magnetic resin beads for the purposes of preventing the magnetically responsive powder from falling off and binding a biologically active component such as antigens, antibodies or enzymes.

The polymer coating on the magnetic resin beads may be carried out by a polymerization method wherein a polymerizable monomer, either dissolved in a solvent or used neat is, together with a usual initiator and cross-linking agent if necessary, impregnated to the magnetically responsive powder layer of the magnetic resin beads, followed by heating for polymerization, or a casting method wherein a polymer dissolved in a solvent is impregnated to the magnetically responsive powder layer of the magnetic resin beads, and then evaporating the solvent. The polymer is thereby impregnated into the magnetically responsive powder layer, and therefore the polymer layer thus coated on the magnetic resin beads has substantially the same thickness as the magnetically responsive powder layer, at a level of from 2 to 30 $\mu$m, preferably from 10 to 20 $\mu$m. The solvent to be used for the polymer coating on the magnetic resin beads has to be a poor solvent or a non-solvent of the thermoplastic resin constituting the magnetic resin beads.

The polymer usually has a number average polymerization degree of from 20 to 5,000, preferably from 10 to 1,000. When a monomer is polymerized together with a cross-linking agent on the surface of the magnetic resin beads to form a polymer coating, the cross-linking agent is adjusted so that the number average polymerization degree between the cross-linkages will be from 1 to 500, preferably from 10 to 50.

The manner for binding a biologically active component such as enzymes, antibodies or antigens, varies depending upon the functional groups of the polymer on the polymer-coated magnetic resin beads thus obtained.

In most cases, the binding or immobilization of the enzymes, antibodies or antigens is conducted in an aqueous solution. Accordingly, the polymer to be coated on the magnetic resin beads should not dissolve or swell in water, and therefore an alcohol-type, ether-type or water-soluble type polymer is not suitable for use. Further, the polymer should not dissolve or swell in a solvent which is used for the activation of the polymer to bind enzymes, antibodies or antigens.

For instance, once hydroxyl groups having been introduced by the modification of the surface layer of the polymer-coated magnetic resin beads, a biologically active component such as enzymes, antibodies or

antigens can be immobilized or bound by a conventional method (e.g. N. Hagi et al, Toyo Soda Kenkyu Hokoku 81, 25 (1981)). In some cases antibodies, antigens or enzymes may be directly reacted to the surface layer for immobilization. Further, a hydrophobic polymer may be coated, and antibodies, antigens or enzymes may be adsorbed thereon for immobilization.

The polymer to be used for the coating may suitably be selected depending upon the conditions for the immobilization of the biologically active component such as antibodies, antigens or enzymes. Now, the polymer will be described in further detail.

1) Antibodies, antigens or enzymes are chemically bound directly on the surface of the polymer-coated magnetic resin beads. In this case, the magnetic resin beads have to be coated with a polymer having functional groups capable of binding such a biologically active component. For instance, when the beads are coated with a polymer having aldehyde groups, they react with amino groups of the antibodies, antigens or enzymes to form linkages of a Schiff base. The treated beads are then reduced by a reducing agent such as $NaBCNH_3$ to obtain stabilized linkages. As the polymer, there may be employed a polymer of a monomer having the formula RCH = CR′-CHO wherein each of R and R′ is a hydrogen atom, an alkyl group, a halogen-substituted alkyl group or a phenyl group, and having a total of from 3 to 10 carbon atoms, preferably from 3 to 6 carbon atoms, such as

$CH_2$=CH-CHO or 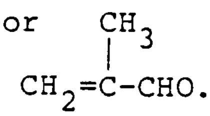

2) Only the surface layer of the polymer-coated magnetic resin beads is treated for the immobilization of antibodies, antigens or enzymes. The manner of the treatment varies depending upon the functional groups on the surface layer. The most common functional groups are hydroxyl groups, which may be activated in accordance with a conventional method (e.g. N. Hagi et al, Toyo Soda Kenkyu Hokoku 81, 25 (1981)). Hydroxy groups may be introduced in various manners. However, it is most convenient to employ a method wherein the magnetic resin beads are coated with a polymer having a repeating unit of the formula:

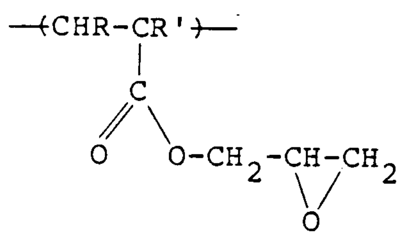

wherein R and R′ are as defined above, and then hydrolyzed by sodium hydroxide in a non-aqueous solvent such as methanol or in a non-aqueous solvent mixture. The hydrophilic magnetic resin beads thus obtained, can further be activated in accordance with a conventional method, as described above.

Various groups are conceivable for R and R′ in the monomer. However, with a view to the prevention of a non-chemical adsorption to the immobilized layer, the monomer preferably has a total of from 6 to 12 carbon atoms.

When amino groups are to be introduced to the surface layer, the above-mentioned epoxy ring is subjected to a ring opening reaction with a diamine having the formula $NH_2$-R″-$NH_2$ wherein R″ is an alkyl group, a halogen-substituted alkyl group or a phenyl group having a total of from 2 to 12, preferably from 2 to 8 carbon atoms. When the treatment is conducted by using ethylene diamine (R″: ethylene), the diamine is added in an amount of more than the stoichiometric amount of the segments of the polymer coated on the magnetic resin beads.

The magnetic resin beads coated with a polymer having epoxy groups may be subjected to partial hydrolysis in accordance with the above-mentioned method. Namely, the partial hydrolysis can be carried out by changing the reaction time or temperature. The partially hydrolyzed polymer-coated beads are reacted with a known silane coupling agent, e.g. of the formula:

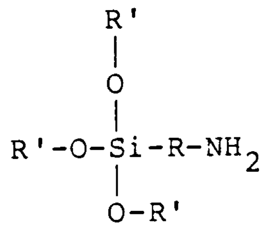

wherein R is an alkyl, aryl or ether group having from 1 to 8 carbon atoms, and R′ is an alkyl or aryl group having from 1 to 8 carbon atoms, which is stable in water or in a non-aqueous solution. In this case, after completion of the reaction, the surface layer will have amino groups and hydroxyl groups in the form of

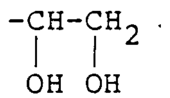 and 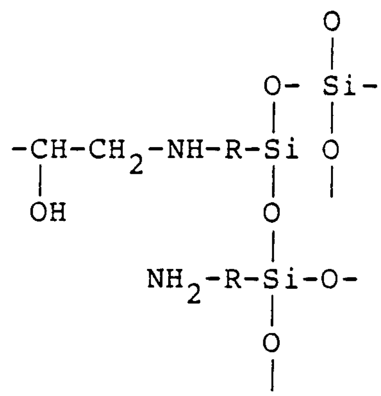

wherein R is as defined above.

3) As other polymers which may be employed for the coating layer of the magnetic resin beads, polymers or copolymers of vinyl chloride, styrene or its derivatives, which are used as the base material for microtiter plates commonly employed in an immunoassay, may be mentioned, although they are not necessarily preferred since they require a plurality of steps for the surface activation. The activation treatment may be conducted as the case requires, in accordance with conventional methods.

Now, the present invention will be described in further detail with reference to Examples. However, it should be understood that the present invention is by no means restricted to these specific Examples.

EXAMPLE 1 (Preparation of resin beads)

4 kg of ethylene-vinyl acetate copolymer pellets (Ultracene 710, trade name, manufactured by Toyo Soda Manufacturing Co., Ltd., melting point: 75 °C) having an average diameter of 1.4 mm and an average length of 1.5 mm obtained by an under water cutting method, were added to 20 liter of an aqueous solution containing 0.5 wt.% of polyvinyl alcohol (polymerization degree: about 500), and mixed and dispersed by a vane-type stirrer. The dispersion is heated by a coil heater to a temperature of 95 °C, and the stirring was continued for one hour. Then, the heating was stopped, and the dispersion was left to cool naturally. After cooling, the resin beads were separated, washed with water and dried. The resin beads thus obtained are spherical with an average diameter of 1.65 mm and has a smooth surface. The yield was 95%.

EXAMPLE 2 (Preparation of resin beads)

35 g of low density polyethylene pellets (Petrocene 356, trade name, manufactured by Toyo Soda Manufacturing Co., Ltd., melting point: 101 °C) having an average diameter of 2 mm and an average length of 2.5 mm obtained by an under water cutting method, were added and dispersed in 50 ml of an aqueous solution containing 3.5% by weight of polyvinyl alcohol (polymerization degree: about 500). The dispersion was dropwise added to 2 liter of polyethylene glycol heated to 115 °C, and after stirring for 15 minutes, left to cool naturally. After cooling, the resin beads were separated, washed with water and dried. The resin beads thus obtained were spherical with an average diameter of 2.5 mm. The yield was about 80%.

EXAMPLE 3 (Preparation of resin beads)

1 kg of ethylene-vinyl acetate copolymer pellets (Ultracene 710, trade name, manufactured by Toyo Soda Manufacturing Co., Ltd., melting point: 70° C) having an average diameter of 1.4 mm and an average length of 1.5 mm obtained by an under water cutting method, were added to 5 liter of an aqueous solution containing 6% by weight of Extran MA (manufactured by Merck Co.), and heated and stirred at 95° C for one hour. After cooling naturally, the resin beads were separated, washed with water and dried. The resin beads thus obtained were spherical with an average diameter of 1.65 mm. The yield was about 95%.

EXAMPLE 4 (Preparation of resin beads)

100 g of polystyrene pellets (Denka Styrol 9p-2, trade name, manufactured by Denki Kagaku Kogyo Kabushiki Kaisha, melting point: 185° C) having an average diameter of 2.0 mm and a length of 3.0 mm prepared by a strand method, were added to 1 liter of polyethylene glycol having 90 g of ferrite (wet-type ferrite, manufactured by Toyo Soda Manufacturing Co., Ltd., particle size: 0.3 µm) dispersed therein, and the mixture was heated and stirred at 200° C for 30 minutes. After rapidly cooling with cool water the resin beads were separated, washed and dried. The resin beads thus obtained were spherical with an average diameter of about 2.6 mm. The yield was 100%.

COMPARATIVE EXAMPLE 1

35 g of the same polyethylene pellets as used in Example 2 were added to 2 liter of polyethylene glycol without being dispersed in polyvinyl alcohol. The dispersion was heated and stirred at 115° C for 15 minutes, and then left to cool naturally, whereby the resin underwent coagulation.

EXAMPLE 5

300 g of spherical beads of an ethylene-vinyl acetate copolymer (EVA) obtained in Example 1 and 60 g of a soft ferrite of the formula $(Mn,Zn)Fe_2O_4$ having an average particle size of 0.3 µm were introduced into a 2 liter flask, and thoroughly mixed at room temperature by means of a rotary evaporator. The mixture was heated under stirring while measuring the temperature of the beads. When the temperature reached 90° C, the heating was stopped, and the mixture was maintained at that temperature for from 15 to 20 minutes, and then gradually cooled. In the magnetic resin beads thus obtained, the ferrite particles are not firmly bonded, and a part of ferrite particles fell off by abrasion. However, the deposited ferrite particles were not completely removed, and 80% by weight of the ferrite particles were found to be fused in the EVA in the thickness of from 5 to 15 µm by the electron microscopic (SEM) observation of the magnetic resin beads. Further, the spherical degree of the beads was found unchanged from the initial spherical degree.

Then, the magnetic resin beads thus obtained were coated with the following polymers.

(1) A solution mixture comprising 15 g of an acrolein monomer, 0.6 g of tetraethyleneglycol dimethacrylate and 0.2 g of benzoylperoxide (BPO) as an initiator, was impregnated to 100 g of the magnetic resin beads under stirring. Then, the mixture was heated to 60° C and polymerized for about 3 hours. After the polymerization, the product was washed with ethanol to remove the unreacted monomers. In this operation, about 5% by weight of ferrite particles were removed.

(2) An acetone solution containing 30% by weight of glycidyl methacrylate (GMA) was prepared. BPO was added as an initiator, and the polymerization was conducted at 60° C for 5 hours. The polymer thus obtained had a number average molecular weight of about 40,000. This polymer solution was diluted with acetone to a concentration of 5% by weight. 30 g of this polyglycidyl methacrylate acetone solution was gradually added to 100 g of the magnetic resin beads under stirring, and acetone was evaporated to conduct the coating of the impregnated polymer. Acetone was further evaporated in vacuum from the polymer-coated magnetic resin beads thus obtained.

Then, the beads thus obtained were treated in the following two methods.

In the first method, the epoxy groups on the surface layer were subjected to a ring opening reaction with a 5 wt.% sodium hydroxide methanol solution to convert the epoxy groups to diols.

In the second method, the epoxy groups on the surface layer were subjected to a ring opening reaction with a 65 wt.% ethylenediamine aqueous solution for 5 hours to introduce amino groups to the surface in the form of

$$-CH(OH)-CH_2-NH-CH_2-CH_2-NH_2$$

100 g of the GMA-coated magnetic resin beads were introduced into an Erlenmeyer flask, and 200 g of a 65 wt.% 1,6-diaminohexane aqueous solution was added and reacted thereto for 12 hours. After the reaction, the beads were washed with water. Amino groups were introduced to the surface in the form of

$$-CH(OH)-CH_2-NH-(CH_2)_6-NH_2 .$$

The product had poor wettability with water as compared with the beads aminated with ethylenediamine.

Further, 100 g of the GMA-coated magnetic resin beads were introduced into an Erlenmeyer flask, and 200 g of a 40 wt.% $NH_2-(CH_2)_{12}-NH_2$ ethanol solution was added and reacted thereto for 12 hours under stirring. Amino groups were introduced to the surface in the form of

$$-CH(OH)-CH_2-NH-(CH_2)_{12}-NH_2 .$$

100 g of the GMA-coated magnetic resin beads were subjected to hydrolysis for about 1 hour in 200 ml of a 5 wt.% sodium hydroxide methanol solution. Then, the beads were treated with a 40 wt.% triethoxy-3-aminopropyl silane aqueous solution, whereby magnetic resin beads having amino groups on their surface were obtained.

The amino groups were detected by a ninhydrin method. Further, the amount of the epoxy groups on the surface was determined by treating the GMA-coated magnetic resin beads with a dimethyl amine aqueous solution, washing them with water until the pH became 7, and titrating with hydrochloric acid for neutralization.

(3) To 100 g of the magnetic resin beads, a monomer solution comprising 11 g of a glycidyl methacrylate monomer, 0.2 g of tetraethylene glycol dimethacrylate and 0.1 g of BPO as an initiator, was impregnated at room temperature under stirring. The mixture was stirred at 60 °C for 2 hours to conduct the polymer coating. The product was washed with ethanol, and then subjected to the same activation treatment as in (2).

EXAMPLE 6

Ethylene-vinyl acetate copolymer pellets (Ultracene 625, tradename, manufactured by Toyo Soda Manufacturing Co., Ltd. melting point: 90 °C) were pulverized by a turbomill (T-400 Test Machine, manufactured by Turbo Kogyo Co.). The particle size distribution of the particles were such that particles of from 24 to 34 mesh constituted 37% by weight. Then, 1 kg of such particles were dispersed in 10 liters of a 2 wt.% polyvinyl alcohol aqueous solution at room temperature under stirring. The temperature was gradually raised, and the mixture was stirred at 95 °C for about 2 hours, and then gradually cooled. When the temperature reached a level of from 35 to 40 °C, the product was taken out, and the polyvinyl alcohol deposited on the surface was decomposed with sodium hypochlorite and removed. After drying, the product was classified to obtain a fraction of from 24 to 34 mesh, which constituted 70% by weight. By such treatment for spherical beads, the classification efficiency was improved, and the particle size distribution became sharp or narrow.

Then, 300 g of the particles were introduced into a Kjeldahl shaped flask, and 30 g of ferrite was added. The mixture was stirred at room temperature by means of an evaporator, and the ferrite was substantially uniformly deposited on the surface of the particles. Then, the ferrite-deposited particles were heated in an oil bath at 110 °C for 15 minutes, and then cooled, and the particles were taken out. Thus, ferrite was coated on the surface of the particles.

To a 30 wt.% glycidyl methacrylate acetone solution, a 0.5 wt.% benzoyl peroxide was added, and the mixture was polymerized at 60 °C for 8 hours. The polymerization was terminated with a small amount of methanol. This coating polymer has a number average molecular weight of 48,000, and its molecular weight distribution (weight average molecular weight/number average molecular weight) was 2.8. This 30 wt.% polyglycidyl methacrylate acetone solution as the reaction solution was diluted with acetone to a concentration of from 1 to 5% by weight.

330 g of the ferrite-coated particles were introduced into a planetary type stirrer, and 150 g of the 5 wt.% polyglycidyl methacrylate acetone solution was sprayed under stirring in an air stream to form a polymer coating. Then, the polymer-coated magnetic resin beads were introduced into an evaporator, and adequately dried in vacuum under stirring at a temperature of 60 °C. The polyglycidyl methacrylate-coated magnetic resin beads were added to 700 g of a 5 wt.% methanol sodium hydroxide solution, and hydrolyzed at room temperature for 8 hours. Then, the beads were adequately washed with water, and then dried. The apparent specific gravity of the beads was from 1.04 to 1.08, and the particle size distribution was the same as immediately after the treatment for spherical beads.

EXAMPLE 7

Polyethylene pellets (Petrocene 356, tradename, manufactured by Toyo Soda Manufacturing Co., Ltd., melting point: 101 °C) was pulverized by the same turbomill as used in Example 6. The particles thus obtained had a particle size distribution such that particles of from 24 to 60 mesh constituted 85% by weight. To 300 g of this particles, 60 g of ferrite (0.3 µm, $(Mn,Zn)Fe_2O_4$) was added, and the mixture was introduced into a 1 liter Kjeldahl shaped flask, and stirred at room temperature by an evaporator to uniformly deposit ferrite on the surface of the polyethylene particles.

Then, the ferrite-deposited particles were gradually heated under stirring to 110 °C, maintained at that temperature for 15 minutes and then gradually cooled. 150 g of the magnetic resin beads thus obtained were treated with 80 g of a THF solution of 5 wt.% polystyrene (Denka Styrol 9D-2, tradename, manufactured by Denki Kagaku Kogyo Kabushiki Kaisha) in the same manner as in Example 5 to form a polystyrene coating on the surface of the beads. No substantial peeling off of ferrite from the polystyrene-coated magnetic resin beads was observed. From the observation of the surface of the beads by SEM, it was found that the surface after the ferrite coating was an irregular surface with ferrite deposited thereon without fusion, whereas the surface after the polystyrene coating, was smooth and had a thickness of from 2 to 15 µm in its cross-section.

Separately, 150 g of the magnetic resin beads were treated with 80 g of a THF solution of 5 wt.% polyvinyl chloride (Ryulon paste, tradename, manufactured by Toyo Soda Manufacturing Co., Ltd.) in the same manner as in Example 5 to form a polyvinyl chloride coating on the surface of the beads. No substantial peeling off of ferrite was observed.

EXAMPLE 8

By using the polymer-coated magnetic resin beads (diameter: about 1.6 mm) obtained in Example 5(3), an enzymatic immunoassay of ferritin was conducted by a sandwich method. Namely, five types of magnetic resin beads i.e. (1) those having alcoholic hydroxyl groups formed by saponification on the surface, (2) those having amino groups formed by treatment with ethylenediamine on the surface, (3) those having amino groups formed by treatment with 1,6-diaminohexane on the surface, (4) those having amino groups formed by treatment with 1,12-diaminododecane on its surface and (5) those having amino groups formed by the treatment with triethoxy-3-aminopropylsilane on its surface, were employed.

Binding of antibodies on beads (1) (CDI method):

Magnetic resin beads (2000 beads) having alcoholic hydroxyl groups were adequately dried, and vigorously stirred with 5.0 ml of dry acetone containing 100 mg of N,N′-carbonyl diimidazole (CDI) in a nitrogen atmosphere at room temperature for 30 minutes. The activated magnetic resin beads thus obtained were washed, and 4.0 ml of a basic buffer solution containing 4.0 mg of anti-ferritin monoclonal antibodies, was added thereto for immobilization. Then, non-specific adsorption portions were blocked with bovine serum albumin (BSA) in accordance with a usual method.

Binding of antibodies on beads (2) to (5) (ethylenediamine method, 1,6-diaminohexane method, 1,12-diaminododecane and silane coupling agent method, respectively):

Magnetic resin beads (2000 beads) having amino groups were treated with a basic buffer solution containing glutaraldehyde at room temperature, and then thoroughly washed to remove excessive glutaraldehyde. Then, 4.0 ml of a basic buffer solution containing 4.0 mg of anti-ferritin monoclonal antibodies, was added thereto for immobilization by the formation of a Schiff base. Then, the Schiff base was reduced by sodium cyanoborohydride to form stable chemical bonds. Then, in the same manner as mentioned above, non-specific adsorption portions were blocked with BSA.

By using another monoclonal antibody capable of forming a sandwich with the above-mentioned immobilized antibodies, an enzymatic immunoassay was conducted by means of antibodies labelled with an alkaline phosphatase (EC 3.1.3.1) derived from bovine intestinal mucosa. Namely, 12 antibody-bound magnetic resin beads were introduced into separate immunoassay reactors, and 20 $\mu$l of a serum containing 0, 20, 50, 100, 200 or 500 ng/ml of ferritin and 125 $\mu$l of the enzyme-labelled antibody solution were added to the respective reactor, whereupon the antigen-antibody reaction was conducted at 37 °C for 40 minutes. After washing and solid-liquid separation (B/F separation), 100 $\mu$l of a 1 mM 4-methylumbelliferyl monophosphate as the substrate for the alkaline phosphatase was added, and the enzyme-substrate reaction was conducted at pH 10.0 at 37 °C for 10 minutes. Then, 2.9 ml of a quenching solution was added to terminate the reaction. The fluorescence of this solution was measured at an excitation wave length of 360 nm and at an emission wave length of 450 nm. The results are shown in Figure 1. The abscissa indicates the concentration of ferritin as the antigen, and the ordinate indicates the fluorescence intensity, whereby the concentration of the formed 4-methylumbelliferone (4MU) is presented by nM unit. In Figure 1,

○: CDI method; ●: 1,6-diaminohexane method;
△: ethylenediamine method;
▲: silane coupling agent method; and
□: 1,12-diaminododecane method.

EXAMPLE 9

By using the magnetic resin beads (diameter: about 1.6 mm) obtained in Example 5(3), a study was made on the binding of the antibodies. As the magnetic resin beads, the same five types as used in Example 8 were employed, and the respective binding of antibodies was conducted in the same manner, except that instead of the antibodies, $^{125}$I-labelled mouse IgG was used for immobilization as a substitute for the monoclonal antibodies. IgG was employed in an amount of 1, 2, 4 and 8 $\mu$g per one magnetic resin bead. The results obtained are shown in Table 1.

Table 1: Amount of bound IgG per magnetic resin bead

| Amount of IgG added | 1 µg/bead | 2 µg/bead | 4 µg/bead | 8 µg/bead |
|---|---|---|---|---|
| CDI method | 0.82 µg | 1.20 µg | 1.56 µg | 1.82 µg |
| Ethylenediamine method | 0.72 µg | 1.08 µg | 1.42 µg | 1.68 µg |
| 1,6-Diaminohexane method | 0.78 µg | 1.16 µg | 1.54 µg | 1.84 µg |
| 1,12-Diaminododecane method | 0.63 µg | 0.96 µg | 1.28 µg | 1.49 µg |
| Silane coupling method | 0.71 µg | 1.04 µg | 1.42 µg | 1.66 µg |

It is evident from Table 1 that the amount of bound IgG and the antigen-antibody reaction (see Figure 1) have good correlation to each other. Namely, this is evident from the comparison of the respective immobilization methods at the amount of IgG added being 2 µg/bead with the results of Figure 1. Further, from the Table, it is seen that each magnetic resin bead is capable of binding only 2 µg of IgG.

EXAMPLE 10

By using the polymer-coated magnetic resin beads (diameter: about 1.6 mm) obtained in Example 5(2) and having amino groups introduced to the surface by 1,6-diaminohexane, enzymatic immunoassays of ferritin, AFP ($\alpha$-fetoprotein) and HCG (human chorionic gonadotropin) were conducted by a sandwich method. For each assay, 2,000 magnetic resin beads obtained in Example 5(2) were subjected to glutaraldehyde treatment and immobilization of antibodies in the same manner as in Example 8. Likewise, alkaline phosphatase-labelled antibodies were prepared in the same manner, and used for the sandwich method for immunoassays. Serums containing the respective antibodies and their amounts of use were as follows. Ferritin: 0, 20, 50, 100, 200, 500 ng/ml (amount used: 200 $\mu$l each); AFP: 0, 25, 50, 100, 200, 300 ng/ml (amount used: 25 $\mu$l each); HCG: 0, 25, 50, 100, 200, 400 mIU/ml (amount used: 50 $\mu$l each). The number of the magnetic resin beads and the amounts of the enzyme-labelled antibody solutions used, were as follows. Ferritin: 12 beads, 125 $\mu$l; AFP: 12 beads, 100 $\mu$l; HCG: 12 beads, 100 $\mu$l, for each assay. The antigen-antibody reaction was conducted at 37°C for 40 minutes, and the enzyme-substrate reaction was conducted at 37°C for 10 minutes. These reactions were conducted in the same manner as in Example 8. The results are shown in Figures 2 to 4. It is evident from each of these Figures that with respect to each of ferritin, AFP and HCG, a good linear relationship is obtained within the range of measurement, and that non-specific adsorption at a 0 concentration is minimum. In the case of ferritin, substantially the same linearity and fluorescence intensity at the respective concentrations as in Example 8 were obtained, thus indicating that the magnetic resin beads obtained in Example 5(2) are substantially the same in their performance as those obtained in Example 5(3).

EXAMPLE 11

By using the magnetic resin beads (diameter: about 1.6 mm) obtained by saponification in Examples 5-(1) and 5(3), enzymatic immunoassays of L-thyroxine ($T_4$) were conducted by a competition method.

The magnetic resin beads obtained in Example 5(1) already had many aldehyde groups on their surface, and anti-$T_4$ monoclonal antibodies were immobilized by utilizing these aldehyde groups in the same manner as described in Example 8 (acrolein method). Likewise, the beads obtained in Example 5(3) already had many primary alcoholic hydroxyl groups on their surface, and the immobilization of antibodies was conducted by a N,N′-carbonyldiimidazole (CDI) method in the same manner as described in Example 8. Here, 200 $\mu$g of the anti-$T_4$ monoclonal antibodies were used for 2,000 magnetic resin beads. Then, in accordance with a known method, $T_4$ was chemically bonded to an alkaline phosphatase to obtain enzyme-labelled antigens. In each immunoassay, eight antibody-bound magnetic resin beads, 130 $\mu$l of the enzyme-labelled antigen solution and 20 $\mu$l of a serum containing various concentrations of $T_4$ ($T_4$ concentration: 0, 4, 10, 25, 65, 75, 160, 400, 1,000 and 10,000 ng/ml were subjected to the antigen-antibody reaction and the enzyme-substrate reaction in the same manner as in Example 8, and the fluorescence intensity was measured. The results are shown in Figure 5. In this Figure, the fluorescence intensity at various concentrations is represented by relative values based on the fluorescence intensity measured at a concentration of $T_4$ of 0 ng/ml by using the magnetic resin beads obtained by CDI method, being set at 100. In this Figure, ●: CDI method; and
○: acrolein method.

It is evident from Figure 5 that when the antibody-immobilized magnetic resin beads obtained by the CDI method are employed, a good competition reaction curve is obtained. Further, it is evident that a non-specific adsorption to the antibody-immobilized magnetic resin beads is minimal according to this method, whereas those obtained by the acrolein method are not so good as those obtained by the CDI method in the reactivity, and certain non-specific adsorption is observed. However, these beads are still adequately useful for the immunoassays.

## Claims

**1.** A carrier for a biologically active component for immunoassay or enzymatic reaction, characterised in that it comprises:

a thermoplastic resin bead having an average diameter of from 0.05 to 20 mm;

from 1 to 25% by weight, based on the weight of the bead, of a magnetically responsive powder bound on the bead; and

a polymer coated thereon to a thickness of from 2 to 30 $\mu$m, the polymer having a number average degree of polymerization of from 20 to 5,000 and having, or being capable of having, functional groups

capable of binding, or being activated to bind, the biologically active component.

2. A carrier as claimed in Claim 1, characterised in that the functional groups of the polymer comprise

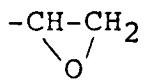, -CHO, -OH or $-NH_2$.

3. A carrier as claimed in Claim 1 or Claim 2, characterised in that the bead is made of a thermoplastic resin selected from a polyolefin resin, a vinyl resin, a polyester resin, a polyamide resin, a copolymer resin, or mixtures thereof.

4. A carrier as claimed in any preceding claim, characterised in that the bead is substantially spherical.

5. A carrier as claimed in any preceding claim, characterised in that the magnetically responsive powder has an average particle size of from 0.01 to 10 μm and is made of iron, tri-iron tetroxide, nickel, iron-cobalt, silicon steel or a soft ferrite of the formula $MFe_2O_4$ wherein M is Mn, Zn, Ni, Cd, Cu, Mg, Sr or Ba.

6. A carrier as claimed in any preceding claim, characterised in that the polymer is a polymer of a monomer having the formula RCH=CR′-CHO wherein each of R and R′ is a hydrogen atom, an alkyl group, a halogen-substituted alkyl group or a phenyl group, and having a total of from 3 to 10 carbon atoms.

7. A carrier as claimed in any of Claims 1 to 5, characterised in that the polymer is a polymer having a repeating unit of the formula:

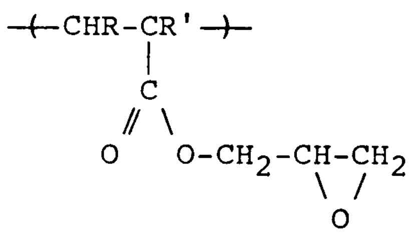

wherein each of R and R′ is a hydrogen atom, an alkyl group, a halogen-substituted alkyl group or a phenyl group, and is obtained from a monomer having a total of from 6 to 12 carbon atoms.

8. A carrier as claimed in any of Claims 1 to 5, characterised in that the polymer is a polymer having -OH groups obtained by hydrolyzing a polymer having a repeating unit of the formula:

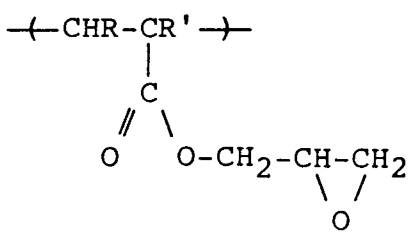

wherein each of R and R′ is a hydrogen atom, an alkyl group, a halogen-substituted alkyl group or a phenyl group, and is obtained from a monomer having a total of from 6 to 12 carbon atoms.

**9.** A carrier as claimed in any of Claims 1 to 5, characterised in that the polymer is a polymer having $-NH_2$ groups obtained by aminating a polymer having a repeating unit of the formula:

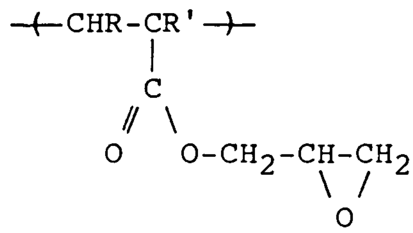

wherein each of R and R′ is a hydrogen atom, an alkyl group, a halogen-substituted alkyl group of a phenyl group, and is obtained from a monomer having a total of from 6 to 12 carbon atoms, with a diamine having the formula $NH_2$-R″-$NH_2$ wherein R″ is an alkyl group, a halogen-substituted alkyl group or a phenyl group having a total of from 2 to 12 carbon atoms.

**10.** A carrier as claimed in any of Claims 1 to 5, characterised in that the polymer is polyglycidyl methacrylate, a glycidyl methacrylate-tetraethyleneglycol dimethacrylate copolymer, an acrolein-tetraethyleneglycol dimethacrylate copolymer, polystyrene or polyvinyl chloride.

**11.** A carrier as claimed in any of Claims 1 to 5, characterised in that the functional groups of the polymer are -OH groups activated by treatment with carbodiimidazole or $-NH_2$ groups activated by treatment with glutaraldehyde.

**12.** A carrier as claimed in any preceding claim, characterised by its having bound to it a biologically active component comprising antigens, antibodies or enzymes.

**13.** A process for preparing the carrier defined by any preceding claim, characterised by depositing the magnetically responsive powder on the surface of the thermoplastic resin bead in an amount of from 1 to 25% by weight, based on the weight of the bead, and forming a layer of the polymer on the bead to a thickness of from 2 to 30 μm.

**14.** A process as claimed in Claim 13, characterised in that the layer of the polymer is formed by polymerizing a monomer having functional groups comprising

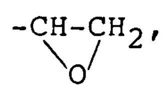

-CHO or -OH to form a polymer having the corresponding functional groups.

**15.** A process as claimed in Claim 14, characterised in that the polymer has

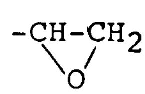

groups and is hydrolyzed to convert the groups to -OH groups or is treated with a diamine to introduce $-NH_2$ groups.

**16.** A process as claimed in Claim 14, characterised in that the polymer has -OH groups and is treated with N,N′-carbonyldiimidazole for activation.

**17.** A process as claimed in Claim 14, characterised in that the polymer has

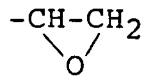

groups and is partially hydrolyzed and reacted with a silane coupling agent having $-NH_2$ groups.

**18.** A process as claimed in Claim 15, characterised in that the polymer has $-NH_2$ groups and is treated with glutaraldehyde for activation.

**19.** A process as claimed in any of Claims 13 to 18, characterised in that the thermoplastic bead is substantially spherical and is prepared by dispersing particles of a thermoplastic resin having a substantially uniform shape in a medium in which the resin is insoluble, at a temperature lower than the melting point of the resin and then heating the dispersion at a temperature of from the melting point of the resin to a temperature not higher than 30 ° C above the melting point.

**20.** A process as claimed in any of Claims 13 to 19, characterised by the additional step of treating the carrier with the biologically active component.

## Revendications

**1.** Support pour un composant biologiquement actif pour immunoessai ou réaction enzymatique, caractérisé en ce qu'il comprend :

une perle de résine thermoplastique ayant un diamètre moyen de 0,05 à 20 mm;

de 1 à 25 % en poids, par rapport au poids de la perle, d'une poudre magnétiquement sensible liée sur la perle; et

un polymère appliqué sur celle-ci d'une épaisseur de 2 à 30 µm, le polymère ayant un degré de polymérisation moyen numérique de 20 à 5.000 et ayant ou pouvant avoir des groupes fonctionnels pouvant lier ou étant activés pour lier le composant biologiquement actif.

**2.** Support suivant la revendication 1, caractérisé en ce que les groupes fonctionnels du polymère comprennent

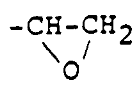 , -CHO, -OH ou $-NH_2$.

**3.** Support suivant l'une ou l'autre des revendications 1 et 2, caractérisé en ce que la perle est faite d'une résine thermoplastique choisie parmi une résine de polyoléfine, une résine vinylique, une résine de polyester, une résine de polyamide, une résine de copolymère ou leurs mélanges.

**4.** Support suivant l'une quelconque des revendications précédentes, caractérisé en ce que la perle est pratiquement sphérique.

**5.** Support suivant l'une quelconque des revendications précédentes, caractérisé en ce que la poudre magnétiquement sensible a une taille de particule moyenne de 0,01 à 10 µm et est faite de fer, de tétroxyde de tri-fer, de nickel, de fer-cobalt, d'acier silicieux ou d'une ferrite douce de la formule $MFe_2O_4$ dans laquelle M représente Mn, Zn, Ni, Cd, Cu, Mg, Sr ou Ba.

**6.** Support suivant l'une quelconque des revendications précédentes, caractérisé en ce que le polymère est un polymère d'un monomère répondant à la formule RCH = CR'-CHO, dans laquelle R et R' représentent chacun un atome d'hydrogène, un groupe alkyle, un groupe alkyle substitué par halogène ou un groupe phényle, et ayant un total de 3 à 10 atomes de carbone.

**7.** Support suivant l'une quelconque des revendications 1 à 5, caractérisé en ce que le polymère est un polymère comportant une unité répétitive de la formule :

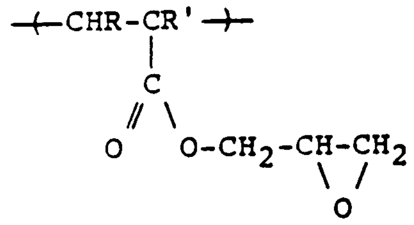

dans laquelle R et R' représentent chacun un atome d'hydrogène, un groupe alkyle, un groupe alkyle substitué par halogène ou un groupe phényle, et est obtenu à partir d'un monomère ayant un total de 6 à 12 atomes de carbone.

**8.** Support suivant l'une quelconque des revendications 1 à 5, caractérisé en ce que le polymère est un polymère comportant des groupes -OH obtenus par hydrolyse d'un polymère comportant une unité répétitive de la formule :

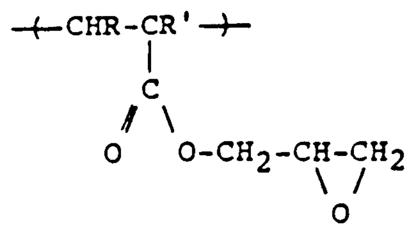

dans laquelle R et R' représentent chacun un atome d'hydrogène, un groupe alkyle, un groupe alkyle substitué par halogène ou un groupe phényle, et est obtenu à partir d'un monomère ayant un total de 6 à 12 atomes de carbone.

**9.** Support suivant l'une quelconque des revendications 1 à 5, caractérisé en ce que le polymère est un polymère comportant des groupes $-NH_2$ obtenus par amination d'un polymère comportant une unité répétitive de la formule :

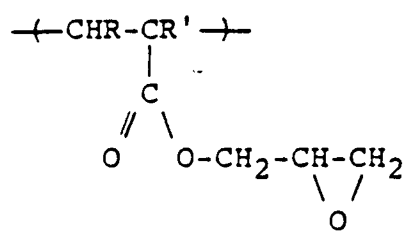

dans laquelle R et R' représentent chacun un atome d'hydrogène, un groupe alkyle, un groupe alkyle substitué par halogène ou un groupe phényle, et est obtenu à partir d'un monomère ayant un total de 6 à 12 atomes de carbone, avec une diamine répondant à la formule $NH_2$-R"-$NH_2$, dans laquelle R" représente un groupe alkyle, un groupe alkyle substitué par halogène ou un groupe phényle ayant un total de 2 à 12 atomes de carbone.

**10.** Support suivant l'une quelconque des revendications 1 à 5, caractérisé en ce que le polymère est du méthacrylate de polyglycidyle, un copolymère de méthacrylate de glycidyle-diméthacrylate de tétraéthylèneglycol, un copolymère d'acroléinediméthacrylate de tétraéthylèneglycol, du polystyrène ou du chlorure de polyvinyle.

**11.** Support suivant l'une quelconque des revendications 1 à 5, caractérise en ce que les groupes fonctionnels du polymère sont des groupes -OH activés par traitement par du carbodiimidazole ou des

groupes $-NH_2$ activés par traitement par du glutaraldéhyde.

**12.** Support suivant l'une quelconque des revendications précédentes, caractérisé en ce qu'il lui est lié un composant biologiquement actif comprenant des antigènes, des anticorps ou des enzymes.

**13.** Procédé de préparation du support défini par l'une quelconque des revendications précédentes, caractérisé par le dépôt d'une poudre magnétiquement sensible sur la surface de la perle de résine thermoplastique en une quantité de 1 à 25 % en poids par rapport au poids de la perle et par la formation d'une couche du polymère sur la perle d'une épaisseur de 2 à 30 µm.

**14.** Procédé suivant la revendication 13, caractérisé en ce que la couche de polymère est formée en polymérisant un monomère comportant des groupes fonctionnels comprenant

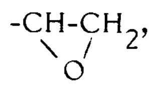

-CHO ou -OH pour former un polymère comportant les groupes fonctionnels correspondants.

**15.** Procédé suivant la revendication 14, caractérisé en ce que le polymère comporte des groupes

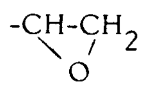

et est hydrolysé pour convertir les groupes en groupes -OH et est traité par une diamine pour introduire des groupes $-NH_2$.

**16.** Procédé suivant la revendication 14, caractérisé en ce que le polymère comporte des groupes -OH et est traité par du N,N'-carbonyldiimidazole pour activation.

**17.** Procédé suivant la revendication 14, caractérisé en ce que le polymère comporte des groupes

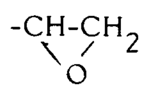

et est partiellement hydrolysé et mis en réaction avec un agent de couplage de silane comportant des groupes $-NH_2$.

**18.** Procédé suivant la revendication 15, caractérisé en ce que le polymère comporte des groupes $-NH_2$ et est traité par du glutaraldéhyde pour activation.

**19.** Procédé suivant l'une quelconque des revendications 13 à 18, caractérisé en ce que la perle thermoplastique est pratiquement sphérique et est préparée en dispersant des particules d'une résine thermoplastique ayant une forme pratiquement uniforme dans un milieu dans lequel la résine est insoluble, à une température inférieure au point de fusion de la résine et ensuite en chauffant la dispersion à une température allant du point de fusion de la résine à une température ne dépassant pas 30°C au-dessus du point de fusion.

**20.** Procédé suivant l'une quelconque des revendications 13 à 19, caractérisé par l'étape de traitement additionnelle du support par le composant biologiquement actif.

**Patentansprüche**

**1.** Träger für eine biologisch aktive Komponente für Immunoassay oder enzymatische Reaktion, dadurch

gekennzeichnet, daß er umfaßt:
Ein thermoplastisches Harzkügelchen mit einem durchschnittlichen Durchmesser von 0,05 bis 20 mm; von 1 bis 25 Gew.-%, bezogen auf das Gewicht des Kügelchens, eines magnetisch ansprechenden Pulvers, das auf dem Kügelchen gebunden ist; und
ein Polymeres, das darauf bis zu einer Dicke von 2 bis 30 µm beschichtet ist, wobei das Polymere ein Zahlenmittel des Polymerisationsgrades von 20 bis 5000 aufweist und funktionelle Gruppen mit der Fähigkeit, die biologisch aktive Komponente zu binden, oder mit der Fähigkeit, dazu aktiviert zu werden, die biologisch aktive Komponente zu binden, aufweist oder in der Lage ist, solche funktionellen Gruppen aufzuweisen.

**2.** Träger gemäß Anspruch 1, dadurch gekennzeichnet, daß die funktionellen Gruppen des Polymeren

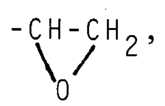

-CHO, -OH oder $NH_2$ umfassen.

**3.** Träger gemäß Anspruch 1 oder Anspruch 2, dadurch gekennzeichnet, daß das Kügelchen aus einem thermoplastischen Harz besteht, das ausgewählt ist aus einem Polyolefinharz, einem Vinylharz, einem Polyesterharz, einem Polyamidharz, einem Copolymerisatharz oder Mischungen derselben.

**4.** Träger gemäß einem der vorstehenden Ansprüche dadurch gekennzeichnet, daß das Kügelchen im wesentlichen sphärisch ist.

**5.** Träger gemäß einem der vorstehenden Ansprüche, dadurch gekennzeichnet, daß das magnetisch ansprechende Pulver eine durchschnittliche Teilchengröße von 0,01 bis 10 µm aufweist und besteht aus Eisen, Trieisentetroxid, Nickel, Eisen-Kobalt, Siliciumstahl oder einem weichen Ferrit der Formel $MFe_2O_4$, wobei M für Mn, Zn, Ni, Cd, (Cu, Mg, Sr oder Ba steht.

**6.** Träger gemäß einem der vorstehenden Ansprüche, dadurch gekennzeichnet, daß das Polymere ein Polymeres von einem Monomeren der Formel RCH=(CR'-CHO ist, wobei jedes von R und R' für ein Wasserstoffatom, eine Alkylgruppe, eine Halogen-substituierte Alkylgruppe oder eine Phenylgruppe steht und eine Gesamtzahl von 3 bis 10 Kohlenstoffatomen aufweist.

**7.** Träger gemäß einem der Ansprüche 1 bis 5, dadurch gekennzeichnet, daß das Polymere ein Polymeres mit Wiederholungseinheiten der folgenden Formel ist:

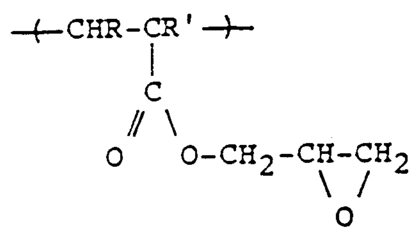

wobei jedes von R und R' für ein Wasserstoffatom, eine Alkylgruppe, eine Halogen-substituierte Alkylgruppe oder eine Phenylgruppe steht, und erhalten wird aus einem Monomeren mit einer Gesamtzahl von 6 bis 12 Kohlenstoffatomen.

**8.** Träger gemäß einem der Ansprüche 1 bis 5, dadurch gekennzeichnet, daß das Polymere ein Polymeres mit OH-Gruppen ist, erhalten durch Hydrolyse eines Polymeren mit einer Wiederholungseinheit der Formel:

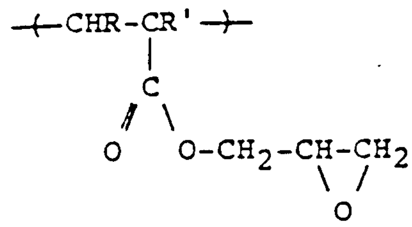

wobei jedes von R und R' für ein Wasserstoffatom, eine Alkylgruppe, eine Halogen-substituierte Alkylgruppe oder eine Phenylgruppe steht, und erhalten wird von einem Monomeren mit einer Gesamtzahl von 6 bis 12 Kohlenstoffatomen.

**9.** Träger gemäß einem der Ansprüche 1 bis 5, dadurch gekennzeichnet, daß das Polymere ein Polymeres mit $NH_2$-Gruppen ist, erhalten durch Aminierung eines Polymeren mit einer Wiederholungseinheit der Formel:

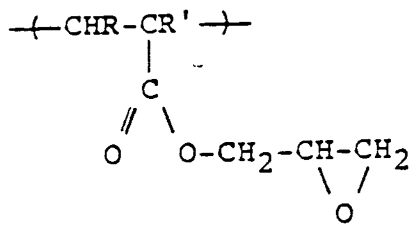

wobei jedes von R und R' für ein Wasserstoffatom, eine Alkylgruppe, eine Halogen-substituierte Alkylgruppe oder eine Phenylgruppe steht, und erhalten wird aus einem Monomeren mit einer Gesamtzahl von 6 bis 12 Kohlenstoffatomen, mit einem Diamin mit der Formel $NH_2$-R''-$NH_2$, wobei R'' für eine Alkylgruppe, eine Halogen-substituierte Alkylgruppe oder eine Phenylgruppe mit einer Gesamtzahl von 2 bis 12 Kohlenstoffatomen steht.

**10.** Träger gemäß einem der Ansprüche 1 bis 5, dadurch gekennzeichnet, daß das Polymere Polyglycidylmethacrylat, ein Glycidylmethacrylat-Tetraethylenglycoldimethacrylatcopolymeres, ein Acrolein-Tetraethylenglycoldimethacrylatcopolymeres, Polystyrol oder Polyvinylchlorid ist.

**11.** Träger gemäß einem der Ansprüche 1 bis 5, dadurch gekennzeichnet, daß die funktionellen Gruppen des Polymeren OH-Gruppen sind, aktiviert durch Behandlung mit Carbodiimidazol, oder-$NH_2$-Gruppen sind, aktiviert durch Behandlung mit Glutaraldehyd.

**12.** Träger gemäß einem der vorstehenden Ansprüche, dadurch gekennzeichnet, daß an ihn eine biologisch aktive Komponente gebunden ist, welche Antigene, Antikörper oder Enzyme umfaßt.

**13.** Verfahren zur Herstellung des Trägers, der durch irgendeinen der vorstehenden Ansprüche definiert ist, dadurch gekennzeichnet, daß man das magnetisch ansprechende Pulver auf der Oberfläche des thermoplastischen Harzkügelchens in einer Menge von 1 bis 25 Gew.-%, bezogen auf das Gewicht des Kügelchens, ablagert und auf dem Kügelchen eine Schicht des Polymeren bis zu einer Dicke von 2 bis 30 µm ausbildet.

**14.** Verfahren gemäß Anspruch 13, dadurch gekennzeichnet, daß die Schicht des Polymeren ausgebildet wird, indem man ein Monomeres mit funktionellen Gruppen, umfassend

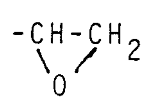

-CHO oder -OH, polymerisiert, um ein Polymeres mit den entsprechenden funktionellen Gruppen zu bilden.

**15.** Verfahren gemäß Anspruch 14, dadurch gekennzeichnet, daß das Polymere

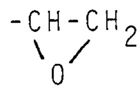

-Gruppen aufweist und hydrolisiert wird, um die Gruppen in -OH-Gruppen umzuwandeln, oder mit einem Diamin behandelt wird, um $-NH_2$-Gruppen einzuführen.

**16.** Verfahren gemäß Anspruch 14, dadurch gekennzeichnet, daß das Polymere -OH-Gruppen aufweist und zur Aktivierung mit N,N'-Carbonyldiimidazol behandelt wird.

**17.** Verfahren gemäß Anspruch 14, dadurch gekennzeichnet, daß das Polymere

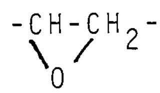

Gruppen aufweist und teilweise hydrolisiert wird und mit einem Silankupplungsmittel umgesetzt wird, welches $-NH_2$-Gruppen aufweist.

**18.** Verfahren gemäß Anspruch 15, dadurch gekennzeichnet, daß das Polymere $-NH_2$-Gruppen aufweist und zur Aktivierung mit Glutaraldehyd behandelt wird.

**19.** Verfahren gemäß einem der Ansprüche 13 bis 18, dadurch gekennzeichnet, daß das thermoplastische Kügelchen im wesentlichen sphärisch ist und hergestellt wird, indem man Teilchen eines thermoplastischen Harzes mit einer im wesentlichen einförmigen Gestalt in einem Medium dispergiert, in dem das Harzunlöslich ist, bei einer Temperatur, die niedriger ist als der Schmelzpunkt des Harzes und anschließend die Dispersion bei einer Temperatur erhitzt, die vom Schmelzpunkt des Harzes bis zu einer Temperatur nicht höher als 30 ° C oberhalb des Schmelzpunktes liegt.

**20.** Verfahren gemäß einem der Ansprüche 13 bis 19, gekennzeichnet durch die zusätzliche Stufe der Behandlung des Trägers mit der biologisch aktiven Komponente.

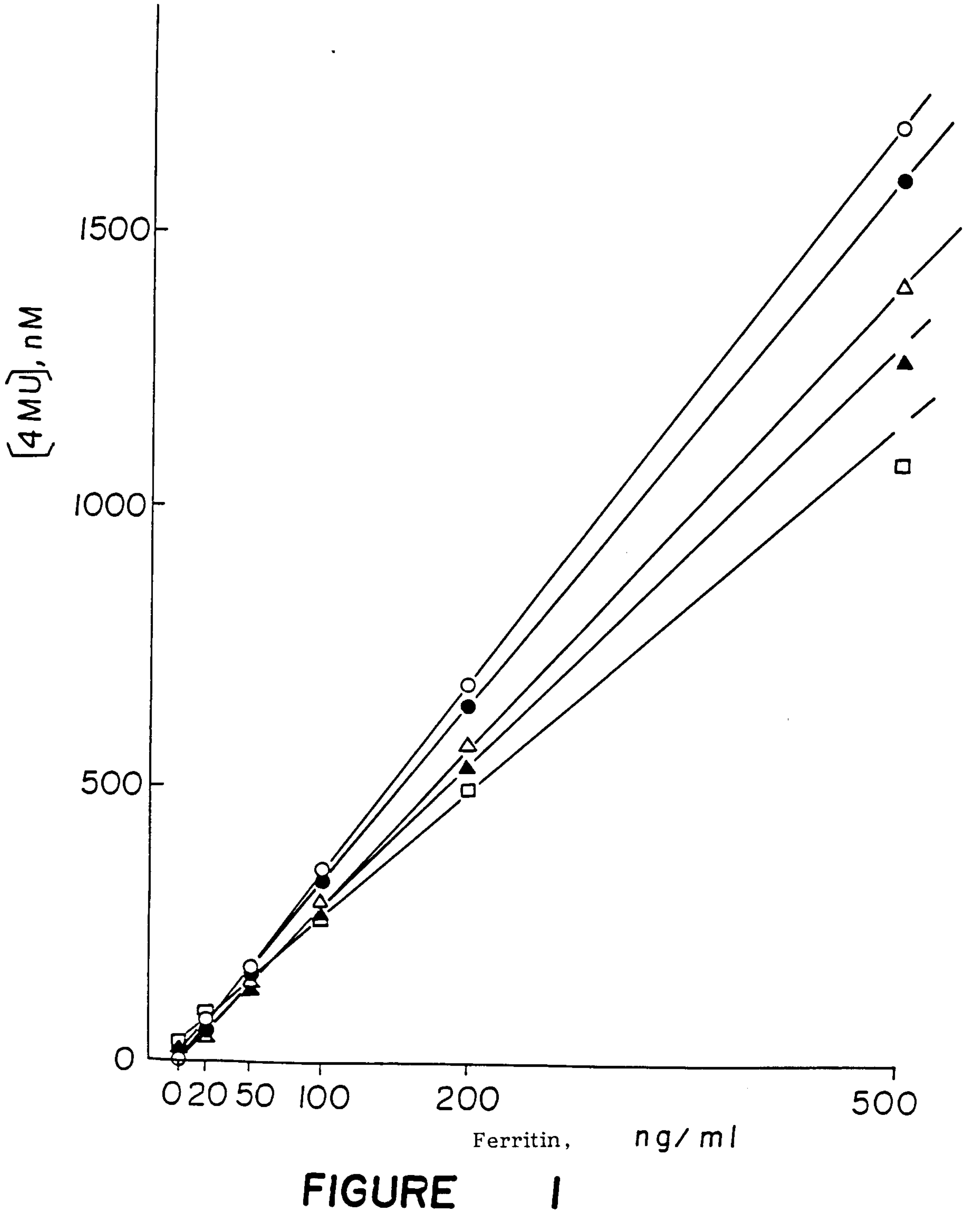


FIGURE 1

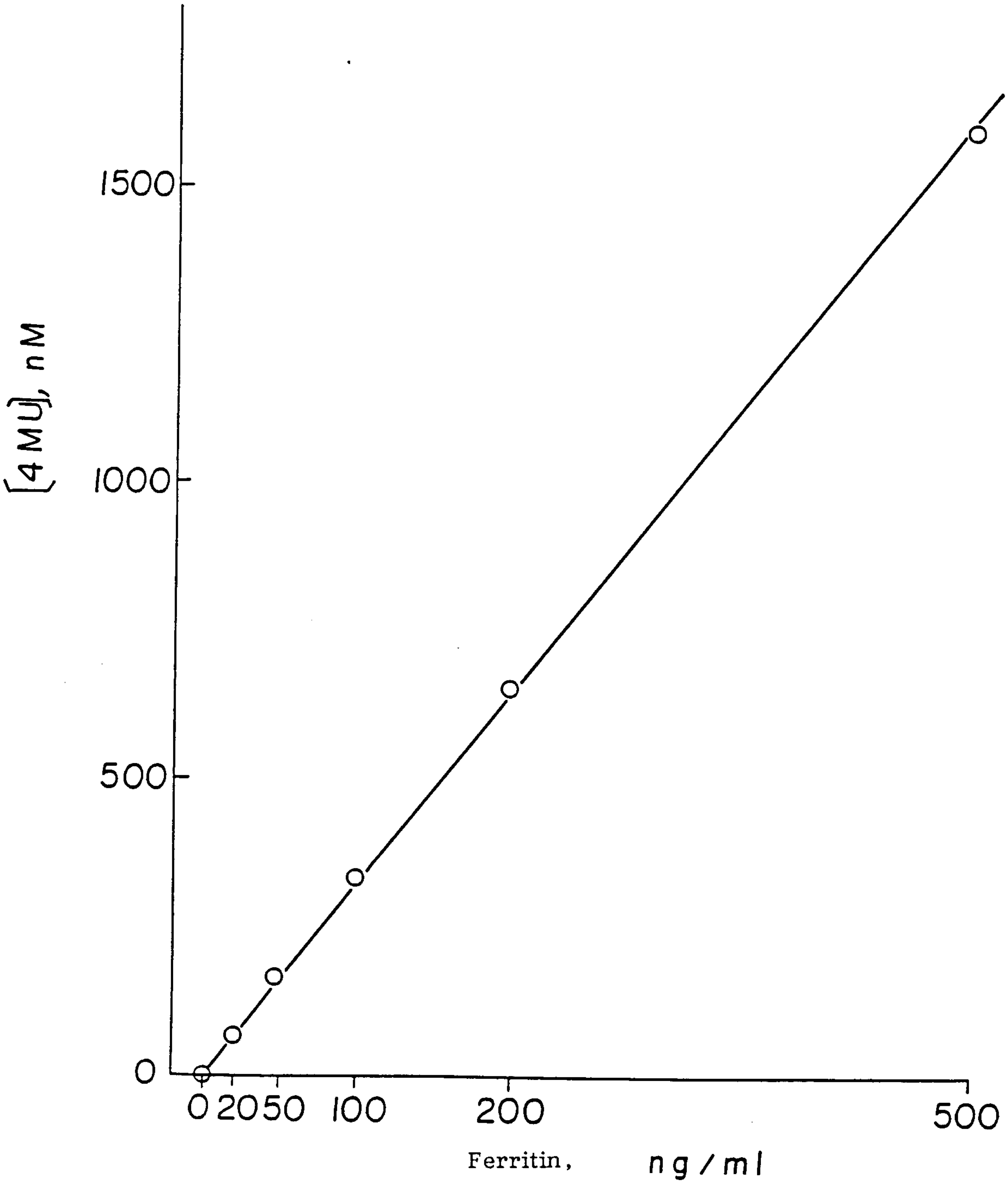


FIGURE 2

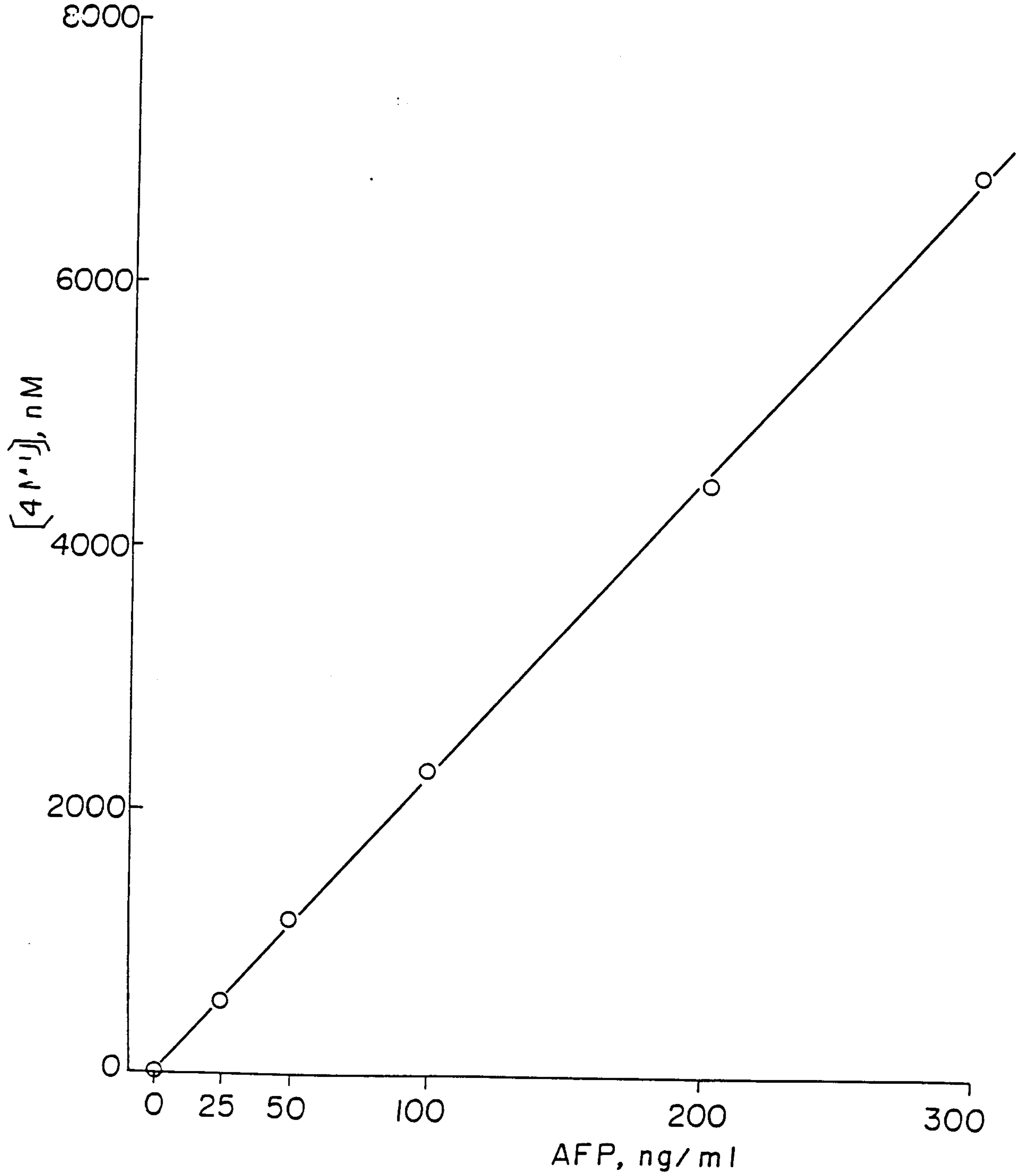


FIGURE 3

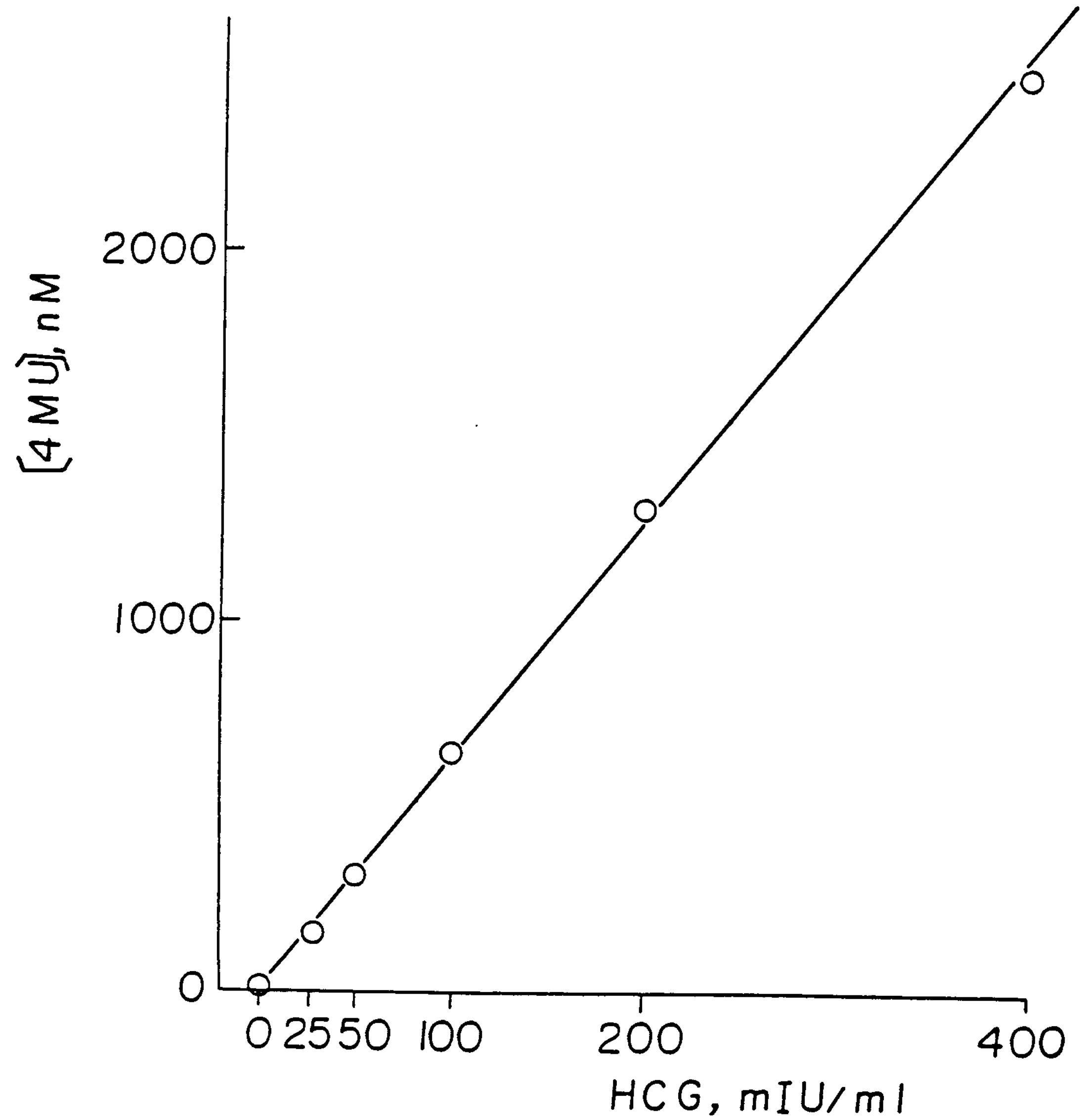


FIGURE 4

Standard Curve of L-Thyroxine

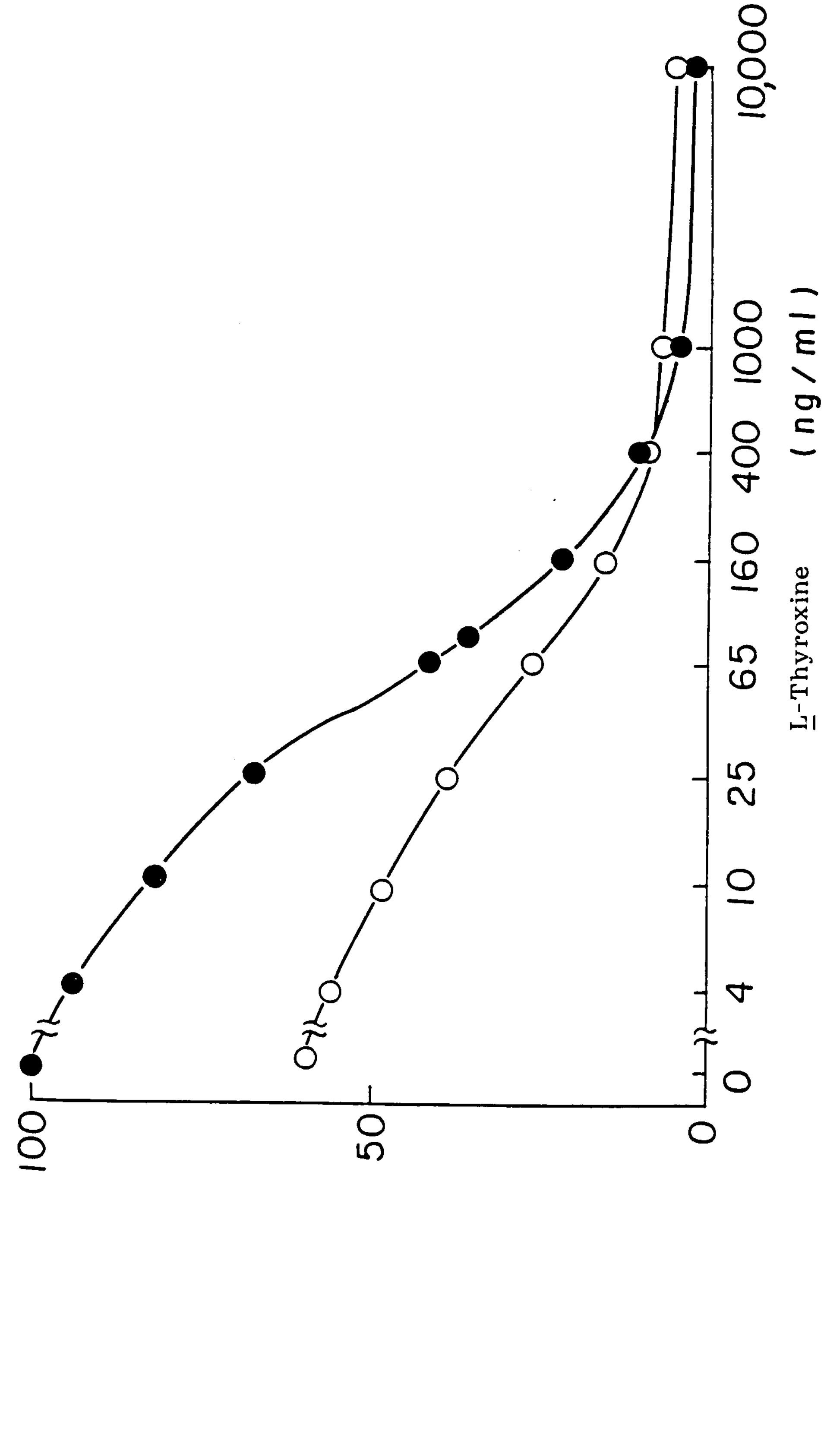


FIGURE 5